# FASCICULE DE BREVET EUROPEEN

(11) **EP 0 573 318 B1**
(45) Date de publication et mention de la délivrance du brevet: **24.03.1999**
(21) Numéro de dépôt: 93401114.9
(22) Date de dépôt: 29.04.1993
(51) Int. Cl.: C07D 261/18, A61K 31/42, C07C 235/80, C07D 295/14, C07C 323/36

(54) **Dérivés de l'acide 4-phénylcarbamoyl-3-isoxazolecarboxylique à activité antiinflammatoire**
4-Phenylcarbamoyl-3-isoxazolecarbonsäure-Derivate mit entzündungshemmender Aktivität
4-Phenylcarbamoyl-3-isoxazolecarboxylic acid derivatives with antiinflammatory activity

(30) Priorité: 30.04.1992 GB 9209330
(43) Date de publication de la demande: 08.12.1993
(73) Titulaire: HOECHST MARION ROUSSEL, 92800 Puteaux (FR)
(72) Inventeur: Bartlett, Robert R., W-6100 Darmstadt 12 (DE); Kuo, Elizabeth Anne, Swindon, Wiltshire (GB); Matharu, Saroop Singh, Cricklade, Wiltshire (GB); Schleyerbach, Rudolf, W-6238 Hofheim (DE); Westwood, Robert, Kingston Bagpuize, Oxon (GB)
(74) Mandataire: Vieillefosse, Jean-Claude

(56) Documents cités:
- EP-A- 0 012 435
- EP-A- 0 326 108
- EP-A- 0 440 503
- FR-A- 2 313 031

## Description

Cette invention concerne des dérivés de l'acide 4-phénylcarbamoyl-3-isoxazolecarboxylique et leurs sels, les procédés de leur préparation, les compositions pharmaceutiques les contenant et leur utilisation comme médicaments.
Des dérivés de N-phényl-4-isoxazolecarboxamide substitués en position 3 et en position 5 et ayant une activité anti-inflammatoire ont été décrits dans la demande de brevet européenne EP 440 503.
La demande de brevet européenne EP 326108 décrit des dérivés de N-phényl-4-isoxazolecarboxamide substitués en position 5 et ayant une activité anti-inflammatoire.
La demande de brevet française FR 2313031 divulgue des composés isoxazoles utilisés comme produits intermédiaires.

Selon un aspect de l'invention, nous proposons les composés de formule (I): 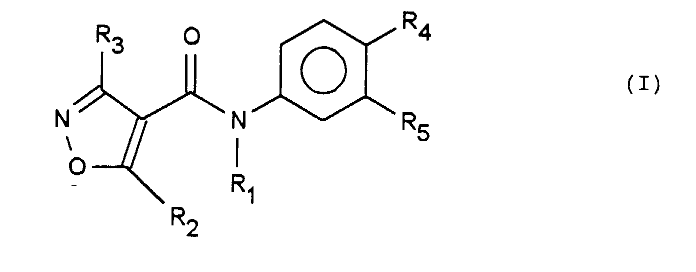 dans lesquels
R₁ représente un atome d'hydrogène ou un groupement alkyle comportant de 1 à 3 atomes de carbone;
R₂ représente un groupement alkyle de 1 à 3 atomes de carbone ou un groupement cycloalkyle comportant de 3 à 6 atomes de carbone;
R₃ représente un groupement -COOH ou un groupement -COOR₆ dans lesquels R₆ représente un groupement alkyle comportant de 1 à 3 atomes de carbone;
R₄ repésente un atome d'halogène, un groupement nitrile, un groupement nitro, un groupement -SCH₃, un groupement alkylcarbonyle en C₁₋₆, un groupement cycloalkylcarbonyle en C₃₋₆, un groupement -CX₃, un groupement -WCX₃, un groupement -(CH₂)ₙCX₃,un groupement -(CX₂)ₙCX₃, un groupement -W(CX₂)ₙCX₃ ou un groupement -W(CH₂)ₙCX₃,
dans lesquels X représente un atome d'halogène, W représente un atome d'oxygène ou de soufre, et n représente 1, 2 ou 3; et
R₅ représente un groupement alkyle comportant de 1 à 3 atomes de carbone ou un groupement cycloalkyle comportant de 3 à 6 atomes de carbone et leurs sels d'addition de base.

Il est entendu que l'invention s'étend à toutes les formes tautomères des composés de formule (I).

Au sens de la présente invention, le terme "groupement cycloalkyle comportant de 3 à 6 atomes de carbone" désigne un groupement cyclopropyle, cyclobutyle, cyclopentyle ou cyclohexyle.

Au sens de la présente invention, le terme "groupement alkyle comportant de 1 à 3 atomes de carbone" désigne un groupement méthyle, éthyle, propyle ou isopropyle.

Au sens de la présente invention, le terme "atome d'halogène" désigne un atome de fluor, de chlore, de brome ou d'iode.

Les sels d'addition de base peuvent être des sels avec les bases minérales ou organiques, par exemple des sels formés avec les bases minérales tels que les sels de sodium, de potassium, de lithium, de calcium, de magnésium et d'ammonium, ou des sels formés avec les bases organiques telles que la méthylamine, la propylamine, la triméthylamine, la diéthylamine, la triéthylamine, la N,N-diméthyléthanolamine, le tris(hydroxyméthyl)aminométhane, l'éthanolamine, la pyridine, la picoline, la dicyclohexylamine, la morpholine, la benzylamine, la procaïne, la lysine, l'arginine, l'histidine et la N-méthylglucamine.

Les composés préférés selon l'invention sont ceux dans lesquels
R₁ représente un atome d'hydrogène,
R₂ représente un groupement méthyle ou un groupement cyclopropyle; R₄ représente un atome d'halogène, un groupement nitrile, un groupement nitro, un groupement -CF₃, un groupement -OCF₅ ou un groupement -SCF₃; et R₅ représente un groupement méthyle ou éthyle, ou un groupement cyclopropyle.

Les composés plus particulièrement préférés selon l'invention sont ceux dans lesquels
R₄ représente un groupement -CF₃, un groupement -OCF₃ ou un groupement -SCF₃ et R₅ représente un groupement méthyle.

Les composés notamment préférés sont:
l'acide 5-méthyl-4-[N-(3-méthyl-4-trifluorométhyl)phényl]carbamoyl-3-isoxazolecarboxylique;
l'acide 5-méthyl-4-[N-(3-méthyl-4-trifluorométhoxy)phényl]carbamoyl-3-isoxazolecarboxylique;
l'acide 5-cyclopropyl-4-[N-(3-méthyl-4-trifluorométhoxy)phényl]carbamoyl-3-isoxazolecarboxylique; et leurs sels d'addition de base.

Les composés selon l'invention peuvent par exemple être préparés selon les procédés suivants, lesquels procédés constituent d'autres aspects de la présente invention:

Les composés de formule (I) dans lesquels R₁, R₂, R₄ et R₅ sont tels que définis ci-dessus et R₃ représente un groupement -COOR₃ dans lequel R₆ est tel que défini ci-dessus, peuvent être par exemple préparés par réaction d'un composé de formule (II) 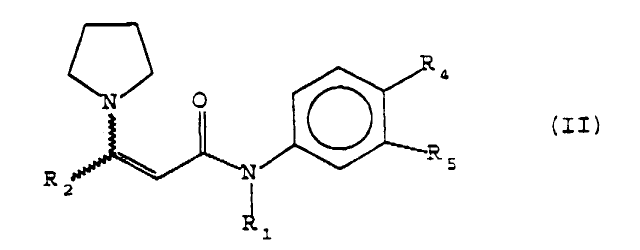 (dans lequel R₁, R₂, R₄ et R₅ sont tels que définis ci-dessus) avec un composé de formule (III) 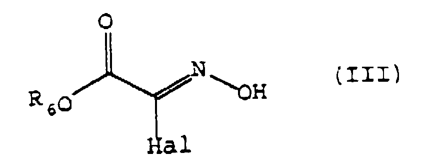 (dans lequel Hal représente un atome d'halogène et R₆ est tel que défini ci-dessus).

La réaction entre le composé de formule (II) et le composé de formule (III) est de préférence effectuée en présence d'un solvant organique anhydre tel que le dichlorométhane ou l'éther diéthylique, et à une température basse.

Pour préparer les composés de formule (I) dans lesquels R₁, R₂ R₄ et R₅ sont tels que définis ci-dessus et R₃ représente un groupement -COOH, les composés ainsi obtenus par la réaction des composés de formules (II) et (III) tels que décrits ci-dessus peuvent être saponifiés.

La saponification peut être effectuée par exemple à l'aide d'acide sulfurique, et en présence par exemple du tétrahydrofurane et de l'eau et à l'aide d'hydroxyde de lithium en présence de méthanol aqueux.

Le composé de formule (II) peut être préparé commodément par réaction d'un composé de formule (IV) 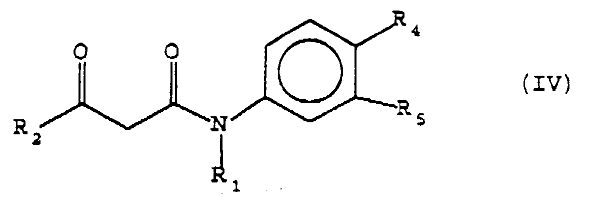 (dans lequel R₁, R₂, R₄ et R₅ sont tels que définis ci-dessus) avec la pyrrolidine.

Le composé de formule (II) peut être préparé comme indiqué ci-dessus et on peut ultérieurement le faire agir in situ (sans isolement) avec le composé de formule (III).

La réaction entre le composé de formule (IV) et la pyrrolidine est de préférence effectuée en présence d'un solvant organique anhydre tel que le benzène ou l'éther et à reflux ou à température ambiante.

Le composé de formule (IV) peut être préparé commodément par réaction d'un composé de formule (V) 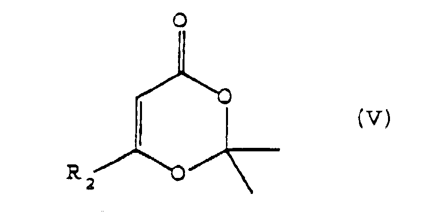 (dans lequel R₂ est tel que défini ci-dessus), avec un composé de formule (VI) 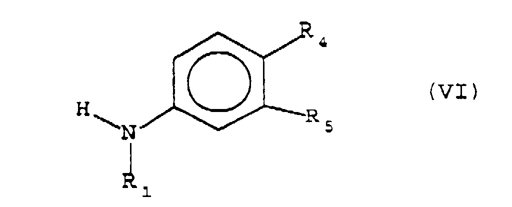 (dans lequel R₁, R₄ et R₅ sont tels que définis ci-dessus).

Ou bien, le composé de formule (IV) peut être préparé commodément par réaction d'un composé de formule (VII)

R₂-COCH₂CO₂Et (VII)

(dans lequel R₂ est tel que défini ci-dessus) avec un composé de formule (VI) tel que défini ci-dessus.

La réaction entre les composés de formules (V) ou (VII) et le composé de formule (VI) est de préférence effectué en présence d'un solvant organique anhydre tel que le xylène ou le toluène, à chaud.

Les composés de formule (VI) mis en oeuvre dans le procédé ci-dessus sont généralement des produits connus ou pouvant être préparés par diazotation, puis réduction des nitroanilines correspondantes selon des procédés connus en soi. Les nitroanilines mises en oeuvre peuvent être préparées comme indiqué par exemple dans TP. Sura et al. Synthetic communications (1988) 18 (16-17) 2161-5. Certaines des anilines de formule (VI) peuvent être préparées selon des procédés décrits dans le brevet européen EP. 206951 ou par réduction des nitrobenzènes correspondants.

Les composés de formule (I) ont un caractère acide lorsque R₃ représente CO₂H. Les sels d'addition de base des composés de formule (I) peuvent être préparés avantageusement par réaction, dans des proportions à peu près stoechiométriques, d'une base minérale ou organique avec le composé de formule (I). Les sels peuvent être préparés sans isolement intermédiaire du composé correspondant.

Les composés selon l'invention présentent des propriétés pharmacologiques intéressantes. D'un intérêt particulier est leur activité anti-inflammatoire remarquable. Ils inhibent à la fois la réponse inflammatoire provoquée par des agents irritants, et les réactions d'hypersensibilité retardées, en génant l'activation des cellules immunitaires par un antigène spécifique.

Ces propriétés sont illustrées plus amplement dans la partie expérimentale.

Les composés de formule (I) et leurs sels d'addition de base sont donc utiles comme médicaments.

Selon un autre aspect de l'invention, on propose l'utilisation en tant que médicament des composés de formule (I) tels que définis ci-dessus et leurs sels d'addition de base pharmacologiquement acceptables.

Les composés préférés selon l'invention pour une utilisation en tant que médicaments sont les composés selon l'invention dans lesquels
R₁ représente un atome d'hydrogène; R₂ représente un groupement méthyle ou un groupement cyclopropyle; R₄ représente un atome d'halogène, un groupement nitrile, un groupement nitro, un groupement -CF₃, un groupement -OCF₃ ou un groupement -SCF₃; et R₅ représente un groupement méthyle ou éthyle, ou un groupement cyclopropyle.

Les composés plus particulièrement préférés selon l'invention pour une utilisation en tant que médicaments sont ceux dans lesquels R₄ représente un groupement -CF₃, un groupement -OCF₃ ou un groupement -SCF₃ et R₅ représente un groupement méthyle.

Les composés notamment préférés selon l'invention pour une utilisation en tant que médicament sont:
l'acide 5-méthyl-4-[N-(3-méthyl-4-trifluorométhyl)phényl]carbamoyl-3-isoxazolecarboxylique;
l'acide 5-méthyl-4-[N-(3-méthyl-4-trifluorométhoxy)phényl]carbamoyl-3-isoxazolecarboxylique;
l'acide 5-cyclopropyl-4-[N-(3-méthyl-4-trifluorométhoxy)phényl]carbamoyl-3-isoxazolecarboxylique;

Un intérêt supplémentaire est présenté par les composés de l'invention dans lesquels R₅ représente un groupement méthyle. Il a été montré que la présence de ce substituant méthyle se traduit par le fait que ces composés présentent une demi-vie métabolique plus courte que les composés correspondants sans substituant méthyle; ceci est illustré plus amplement dans la partie expérimentale. Par conséquent, de tels composés dans lesquels R₅ représente un groupement méthyle représentent des exemples supplémentaires des composés particulièrement préférés selon l'invention pour une utilisation en tant que médicaments.

Ces médicaments sont utiles par exemple dans le traitement de la polyarthrite rhumatoïde et des maladies inflammatoires chroniques d'origine immunitaires ou non-immunitaires (par exemple la maladie homologue, les réactions résultant de la transplantation, uvéite, le psoriasis, le cancer).

La dose habituelle varie selon le composé utilisé, le patient traité et la maladie en question et peut être par exemple de 0,1 mg à 200 mg par jour par voie orale.

Selon un autre aspect de l'invention, on propose des compositions pharmaceutiques comportant à titre de principe actif au moins un composé de formule (I) tel que défini ci-dessus ou un sel d'addition de base pharmaceutiquement acceptable en association avec un ou plusieurs supports et/ou excipients pharmaceutiques.

Pour une utilisation en tant que médicaments, les composés de formule (I) et leurs sels d'addition de base peuvent être incorporés dans des compositions pharmaceutiques destinées à l'administration par voie orale, rectale ou parentérale.

Les compositions pharmaceutiques peuvent être par exemple solides ou liquides et peuvent être sous des formes habituellement utilisées en médecine humaine telle que:
des comprimés, des dragées, des capsules (dont des gélules), des granulés, des suppositoires, des solutions par exemple pour injection; elles peuvent être préparées selon des méthodes classiques. Le(s) principe(s) actif(s) peut (peuvent) être incorporé(s) avec des excipients habituellement utilisés dans des compositions pharmaceutiques telles que le talc, la gomme arabique, l'amidon, la lactose, le stéarate de magnésium, le beurre de cacao, les véhicules aqueux ou non aqueux, les matières grasses d'origine animale ou végétale, les dérivés de la paraffine, les glycols, divers agents mouillants, de dispersion ou émulsifiants et des conservateurs.

Selon un autre aspect de l'invention, on propose un procédé de traitement de la polyarthrite rhumatoïde et des maladies inflammatoires chroniques d'origine immunitaires ou non immunitaires chez un sujet humain ou animal comprenant l'administration au sujet d'une quantité efficace d'un composé de formule (I) tel que défini ci-dessus ou de son sel d'addition de base pharmacologiquement acceptable.

Les composés de formule (II) tels que définis ci-dessus sont eux-mêmes nouveaux et constituent un autre aspect de la présente invention. Ils sont utiles en tant que nouveaux produits industriels et en particulier en tant qu'intermédiaires dans la préparation des composés de formule (I).

L'invention est illustrée plus en détails par les exemples suivants. Il est souligné que seuls les composés où le groupement phényle est disubstitué font partie de l'invention.

### EXEMPLES

### Exemple 1: L'acide 5-méthyl-4-[N-(4-nitrophényl)]carbamoyl-3-isoxazolecarboxylique.

### Etape A

### Préparation de N-(4-nitrophényl)-3-oxobutanamide

La 4-nitroaniline (13,8 g; 0,1 mole) a été mise en suspension dans du xylène anhydre (20 ml) et 2,2,6-triméthyl-4H-1,3-dioxin-4-one(14,22 g; 0,1 mole) ont été ajoutés. Le mélange réactionnel a été chauffé dans un bain d'huile à 150°C pendant 30 minutes tout en éliminant par distillation l'acétone formée au cours de la réaction. Le mélange réactionnel a été refroidi à température ambiante, filtré et le résidu lavé abondamment à l'acétate d'éthyle. Le filtrat a été évaporé pour donner le N-(4-nitrophényl)-3-oxobutanamide (11,65 g; 52 %) sous forme de solide blanc cristallin, F = 119-21°C (à partir de l'acétate d'éthyle - éther de pétrole (40°C-60°C)) (lit. F = 120°-122°C).
* R.J. Clemens et J.A. Hyatt JOC; 50, 2431-35 (1985).

### Etape B

### Préparation de N-(4-nitrophényl)-3-pyrrolidyl-2-buténamide

Un mélange de N-(4-nitrophényl)-3-oxobutanamide (11,10 g; 50 mmole) et de pyrrolidine (4,26 g; 60 mmole) dans du benzène (125 ml) a été chauffé au reflux pendant 1 h à l'aide d'un piège de Dean-Stark pour séparer l'eau. La réaction a été refroidie pour donner le N-(4-nitrophényl)-3-pyrrolidyl-2-buténamide (12,89 g; 94 %) sous forme d'un solide jaune.

### Etape C

### Préparation de 5-méthyl-4-[N-(4-nitrophényl)]carbamoyl-3-isoxaxolecarboxylate d'éthyle (Exemple 14)

Une suspension du N-(4-nitrophényl)-3-pyrrolidyl-2-buténamide brute (12,38 g; 45 mmole) dans du dichlorométhane anhydre (200 ml) a été refroidie à 0°C et du chlorooximidoacétate d'éthyle (7,50 g; 49,5 mmole) a été ajoutée en une fois. Le mélange réactionnel a été agité à -5°C-0°C pendant 2 h et puis versé sur de l'eau (200 ml). La couche aqueuse a été extraite au dichlorométhane (100 ml) et l'extrait organique réuni lavé à l'HCl à 10 % (100 ml), à une solution de NaHCO₃ à 5 % et puis séchée sur MgSO₄. L'évaporation du solvant a donné le 5-méthyl-4-[N-(4-nitrophényl)]carbamoyl-3-isoxazolecarboxylate d'éthyle (10,91 g; 76 %) sous forme de solide blanc cristallin, F = 151-153°C (à partir de l'acétate d'éthyle); IR (KBr) 3120, 1700, 1670, 1620, 1580, 1505, 1335, 1215, 1025, 850 et 750 cm⁻¹; RMN (CDCl₃) δ 11,62 (1H, s, -NH), 8,24 (2H, dd, J = 2,2 Hz et 7,2 Hz, ArH), 7,88 (2H, dd, J = 2,2 Hz et 7,2 Hz, ArH), 4,61 (2H, q, J = 7,2 Hz, -CH₂) 2,89 (3H, s, -CH₃), 1,53 (3H, t, J = 7,2 Hz, -CH₂CH₃).

### Etape D

### Préparation de l'acide 5-méthyl-4-[N-(4-nitrophényl)]carbamoyl-3-isoxazolecarboxylique

Une solution agitée de 5-méthyl-4-[N-(4-nitrophényl)carboxylate d'éthyle (7,98 g; 25 mmole) dans du THF (125 ml) a été traitée avec une solution concentrée d'H₂SO₄ (7,5 ml) dans de l'eau (50 ml). La solution a été chauffée à 50°C pendant 48 h et puis refroidie. L'évaporation du THF sous pression réduite a donnée l'acide 5-méthyl-4-[N-(4-nitrophényl)]carbamoyl-3-isoxazolecarboxylique (4,58 g; 63 %) sous forme de solide blanc cristallin, F = 260-1°C (déc.) (à partir de l'acétate d'éthyle); IR (KBr) 3600-2700, 1715, 1675, 1625, 1575, 1505, 1335 et 1105 cm⁻¹; RMN (DMSO-d₆) δ 11,45 (1H, s, -NH), 8,33 (2H, dd, J = Hz et 9,2 Hz, ArH), 7,92 (2H, dd, J = 2 Hz et 9,2 Hz, ArH), 6,50-4,00 (1H, bs, -CO₂H), 2,67 (3H, s, -CH₃).

En opérant comme à l'exemple 1, on obtient les composés suivants:

### Exemple 2: L'acide 4-[N-(4-cyanophényl)]carbamoyl-5-méthyl-3-isoxazolecarboxylique;

### Exemple 3: L'acide 4-[N-(4-bromo-3-méthyl)phényl]carbamoyl-5-méthyl-3-isoxazolecarboxylique;

### Exemple 4: L'acide 4-[N-(4-chloro-3-méthyl)phényl]carbamoyl-5-méthyl-3-isoxazolecarboxylique;

### Exemple 5: L'acide 5-méthyl-4-[N-(3-méthyl-4-trifluorométhyl)phényl]carbamoyl-3-isoxazolecarboxylique;

### Exemple 6: L'acide 4-[N-(4-cyano-3-méthyl)phényl]carbamoyl-5-méthyl-3-isoxazolecarboxylique;

### Exemple 7: L'acide 5-méthyl-4-[N-(4-trifluorométhylthio)phényl]carbamoyl-3-isoxazolecarboxylique;

### Etape A

### Préparation de N-[4-(trifluorométhylthio)phényl]-3-oxobutanamide

Un mélange de 4-(trifluorométhylthio)aniline (7,72 g; 40 mmole) et de l'acéto-acétate d'éthyle (5,2 g; 40 mmole) dans du toluène anhydre (80 ml) a été chauffé au reflux pendant 8 h à l'aide d'un piège de Dean-Stark pour éliminer l'éthanol formé pendant la réaction. Le mélange réactionnel a été refroidi dans un bain de glace pour donner le N-[4-(trifluorométhylthio)phényl]-3-oxobutanamide (10,48 g; 95 %) sous forme de solide blanc cristallin, F = 120-2°C.

### Etape B

### Préparation de 3-pyrrolidyl-N-[4-trifluorométhylthio]phényl]-2-buténamide

Un mélange de N-[4-(trifluorométhylthio)phényl]3-oxobutanamide (11,08 g; 40 mmole) et de pyrrolidine (3,41 g; 48 mmole) dans du benzène (100 ml) a été chauffé au reflux pendant 1 h à l'aide d'un piège de Dean-Stark pour séparer l'eau. Le mélange réactionnel était refroidi dans un bain de glace pour donner le 3-pyrrolidyl-[N-(4-(trifluorométhylthio)phényl]-2-buténamide (12,57 g; 95 %) sous forme de solide blanc cristallin.

### Etape C

### Préparation de 5-méthyl-4-[N-4-trifluorométhylthio)phényl]carbamoyl-3-isoxazolecarboxylate d'éthyle (Exemple 20)

Une suspension de 3-pyrrolidyl-N-[4-(trifluorométhylthio)phényl]-2-buténamide brute (11,55 g; 35 mmole) dans du dichlorométhane anhydre (150 ml) a été refroidie à 0°C et du chlorooximidoacétate d'éthyle (6,67 g; 44 mmole) a été ajouté. La réaction a été agitée à 0°C pendant 2 h et puis versée sur de l'eau (200 ml). La couche organique a été séparée et la couche aqueuse extraite au dichlorométhane (100 ml). L'extrait organique réuni a été lavé à l'HCl à 10 % (100 ml), à une solution de NaHCO₃ à 5 % (50 ml) et puis séché sur MgSO₄. L'évaporation du solvant a donné le 5-méthyl-4-[N-(4-trifluorométhylthio)phényl]carbamoyl-3-isoxazolecarboxylate (9,74 g; 74 %) sous forme de solide blanc cristallin, F = 117-8°C (à partir d'éther/éther de pétrole (40°-60°)), IR (KBr) 3120, 1710, 1680, 1615, 1325, 1210 et 1135 cm⁻¹; RMN (CDCl₃) δ 11,32 (1H, s, -NH), 7,79 (2H, d, J = 8,8 Hz, ArH), 7,64 (2H, d, J = 8,8 Hz, ArH), 4,59 (2H, q, J = 7,2 Hz, -CH₂), 2,88 (3H, s, -CH₃), 1,51 (3H, t, J = 7,2 Hz, -CH₂CH₃).

### Etape D

### Préparation de l'acide 5-méthyl-4-[N-(4-trifluorométhylthio)phényl]carbamoyl-3-isoxazolecarboxylique

Une solution de monohydrate d'hydroxyde de lithium (1,85 g; 44 mmole) dans de l'eau (25 ml) a été diluée au méthanol (250 ml) et refroidie à -15°C. Le 5-méthyl-4-[N-(4-trifluorométhylthio)phényl]carbamoyl-3-isoxazolecarboxylate d'éthyle (7,48 g; 20 mmole) a été ajouté en petites portions à la solution d'hydroxyde de lithium et le mélange résultant agité à -15°C pendant 5 h, période au cours de laquelle de l'eau (75 ml) a été ajoutée en petites portions. Le mélange réactionnel a été filtré, le filtrat dilué avec de l'eau (400 ml) et acidifié avec du HCl à 2 N pour donner l'acide 5-méthyl-4-[N-(4-trifluorométhylthio)phényl]carbamoyl-3-isoxazolecarboxylique (6,73 g; 97 %) sous forme de solide blanc, F = 158-9°C (déc.) (à partir d'éther/éther de pétrole (40°-60°)); IR (KBr) 3600-2400, 1720, 1680, 1620, 1135 et 1110 cm⁻¹; RMN(DMSO-d₆) δ 11,08 (1H, s, -NH), 7,84 (1H, d, J = 8,6 Hz, ArH), 7,76 (1H, d, J = 8,6 Hz, ArH), 2,66 (3H, s, -CH₃).

En opérant comme à l'exemple 7, les composés suivants sont obtenus:

### Exemple 8: L'acide 5-méthyl-4-[N-(3-méthyl-4-trifluorométhoxy)phényl]carbamoyl-3-isoxazolecarboxylique;

### Exemple 9: L'acide 5-cyclopropyl-4-[N-(4-trifluorométhyl)phényl]carbamoyl-3-isoxazolecarboxylique;

### Etape A

### Préparation de 3-cyclopropyl-3-oxo-N-[4-(trifluorométhyl)phényl]propanamide

Un mélange de 3-cyclopropyl-3-oxopropanoate d'éthyle (10,14 g; 65 mmole) et de 4-aminobenzotrifluoride (10,47 g; 65 mmole) dans du toluène anhydre (100 ml) a été chauffé au reflux pendant 8 h a l'aide d'un condenseur de Dean-Stark pour éliminer l'éthanol formé au cours de la réaction. Le mélange réactionnel a été refroidi et l'éther de pétrole (E = 40°-60°C) ajouté pour donner le 3-cyclopropyl-3-oxo-N-[4-(trifluorométhyl)phényl]-propanamide (16,44 g; 93 %) sous forme de solide blanc cristallin, F = 131-2°C.

### Etape B

### Préparation de 5-cyclopropyl-4-[N-(4-trifluorométhyl)phényl]carbamoyl-3-isoxazolecarboxylate d'éthyle (Exemple 22)

Un mélange de 3-cyclopropyl-3-oxo-N-[4-(trifluorométhyl)phényl]propanamide (13,55 g; 50 mmole), du chlorure de calcium fondu, en grains (8-16 mesh; 6,5 g) et de pyrrolidine anhydre (3,55 g; 50 mmole) dans de l'éther anhydre (350 ml) a été agité à température ambiante pendant 3 h. Le produit résultant, 3-cyclopropyl-3-pyrrolidyl-N-[(4-trifluoroinéthyl)phényl]-2-propénamide n'a pas été isolé mais mis en réaction ultérieurement in situ. Le mélange réactionnel a été refroidi à 0°C et du chlorooximidoacétate d'éthyle (11,37 g; 75 mmole) a été ajouté en une fois suivi de triéthylamine anhydre (7,58 g; 75 mmole) dans de l'éther anhydre (10 ml) ajouté goutte-à-goutte sur 10 min. Le mélange a été agité à 0°C pendant 2 h et puis tiédi à température ambiante. Après 30 min. d'agitation, le mélange réactionnel a été versé sur de l'eau (200 ml), la couche organique séparée et la couche aqueuse extraite par de l'éther (100 ml). L'extrait organique réuni a été lavé à l'HCl à 10 % (100 ml), à une solution de NaHCO₃ à 5 % (100 ml), séché sur MgSO₄ et enfin évaporé à sec. La purification du résidu par chromatographie flash (Kiesegel 60H, 120 g; CH₂Cl₂) a donné le 5-cyclopropyl-4-[N-(4-trifluorométhyl)phényl]carbamoyl-3-isoxazole-carboxylate d'éthyle (12,54 g; 68 %) sous forme de solide blanc cristallin, F = 100-1°C (à partir d'éther de pétrole (40°-60°C)); IR (KBr) 3090, 1710, 1670, 1605, 1325, 1110 et 845 cm⁻¹; RMN CDCl₃ δ 11,31 (1H,s, -NH), 7,85 (2H, d, J = 8,6 Hz), 7,61 (2H, d, J = 8,6 Hz, ArH), 4,57 (2H, q, J = 7,1 Hz, -CH₂), 3,38-3,24 (1H, m, cyclopropyl-H), 1,50 (3H, t, J = 7,1 Hz, -CH₃), 1,40-1,23 (4H, m, cyclopropyl-H).

### Etape C

### Préparation de l'acide 5-cyclopropyl-4-[N-(4-trifluorométhyl)phényl]carbamoyl-3-isoxazolecarboxylique

Une solution de monohydrate d'hydroxyde de lithium (2,14 g; 51 mmole) dans de l'eau (25 ml) a été diluée avec du méthanol (75 ml) et refroidie à -15°C. Une solution de 5-cyclopropyl-4-[N-(4-trifluorométhyl)phényl]carbamoyl-3-isoxazolecarboxylate (8,46 g; 23 mmole) dans du méthanol (200 ml) a été ajoutée sur 20 min. à la solution d'hydroxyde de lithium et le mélange résultant a été agité à -20°C à -15°C pendant 1 h. Le mélange a été filtré, le filtrat dilué avec de l'eau (400 ml) et acidifié à l'aide d'HCl à 2 N pour donner l'acide 5-cyclopropyl-4-[N-(4-trifluorométhyl)phényl]carbamoyl-3-isoxazolecarboxylique (6,89 g; 88 %) sous forme de solide blanc, F = 160-1°C (déc.) (à partir d'éther/éther de pétrole (40°-60°)); IR (KBr) 3600-2300, 1715, 1635, 1600, 1565, 1325 et 1120 cm⁻¹; RMN (DMSO-d₆) δ 11,13 (1H, s, -NH), 7,90 (2H, d, J = 8,6 Hz, ArH), 7,78 (2H, d, J = 8,6 Hz, ArH), 2,60-2,49 (1H, m, cyclopropyl-H), 1,31-1,13 (4H, m, cyclopropyl-H).

En opérant comme à l'exemple 9, les composés suivants ont été obtenus:

### Exemple 10: L'acide 5-cyclopropyl-4-[N-(3-méthyl-4-trifluorométhyl)phényl]carbamoyl-3-isoxazolecarboxylique;

### Exemple 11: L'acide 5-cyclopropyl-4-[N-(4-trifluorométhoxy)phényl]carbamoyl-3-isoxazolecarboxylique;

### Exemple 12: L'acide 5-cyclopropyl-4-[N-(4-trifluorométhylthio)phényl]carbamoyl-3-isoxazolecarboxylique;

### Exemple 13: L'acide 5-cyclopropyl-4-[N-(3-methyl-4-trifluoromethoxy)phényl]carbamoyl-3-isoxazolecarboxylique;

En opérant comme à l'exemple 1 étape C (exemple 14), les esters éthyliques des composés des exemples 2 à 6 ont été obtenus.

### Exemple 15: Le 4-[N-(4-cyanophényl)]carbamoyl-5-méthyl-3-isoxazolecarboxylate d'éthyle.

### Exemple 16: Le 4-[N-(4-bromo-3-méthyl)phényl]carbamoyl-5-méthyl-3-isoxazolecarboxylate d'éthyle.

### Exemple 17: Le 4-[N-(4-chloro-3-méthyl)phényl]carbamoyl-5-méthyl-3-isoxazolecarboxylate d'éthyle.

### Exemple 18: Le 5-méthyl-4-[N-(3-méthyl-4-trifluorométhyl)phényl]carbamoyl-3-isoxazolecarboxylate d'éthyle.

### Exemple 19: Le 4-[N-(4-cyano-3-méthyl)phényl]carbamoyl-5-méthyl-3-isoxazolecarboxylate d'éthyle.

En opérant comme à l'exemple 7 étape C (exemple 20) l'ester éthylique du composé de l'exemple 8 a été obtenu.

### Exemple 21: Le 5-méthyl-4-[N-(4-trifluorométhoxy)phényl]carbamoyl-3-isoxazolecarboxylate d'éthyle.

En opérant comme à l'exemple 9 étape B (exemple 22) les esters éthyliques des composés 10 à 13 ont été obtenus.

### Exemple 23: Le 5-cyclopropyl-4-[N-(3-méthyl-4-trifluorométhyl)phényl]carbamoyl-3-isoxazolecarboxylate d'éthyle.

### Exemple 24: Le 5-cyclopropyl-4-[N-(4-trifluorométhoxy)phényl]carbamoyl-3-isoxazolecarboxylate d'éthyle.

### Exemple 25: Le 5-cyclopropyl-4-[N-(4-trifluorométhylthio)phényl]carbamoyl-3-isoxazolecarboxylate d'éthyle.

### Exemple 26: Le 5-cyclopropyl-4-[N-(3-méthyl-4-trifluorométhoxy)phényl]carbamoyl-3-isoxazolecarboxylate d'éthyle.

Les données spectrales, les rendements, les points de fusion, et les données analytiques pour les exemples 1 à 26 sont présentées dans le tableau I.

### Exemple 27:

Des comprimés ont été préparés selon la formulation suivante:

| | |
|---|---|
| Composé de l'exemple 1 | 20 mg |
| Excipient pour un comprimé qsp | 150 mg |
| (précisions sur l'excipient: lactose, amidon, talc, stéarate de magnésium). | |

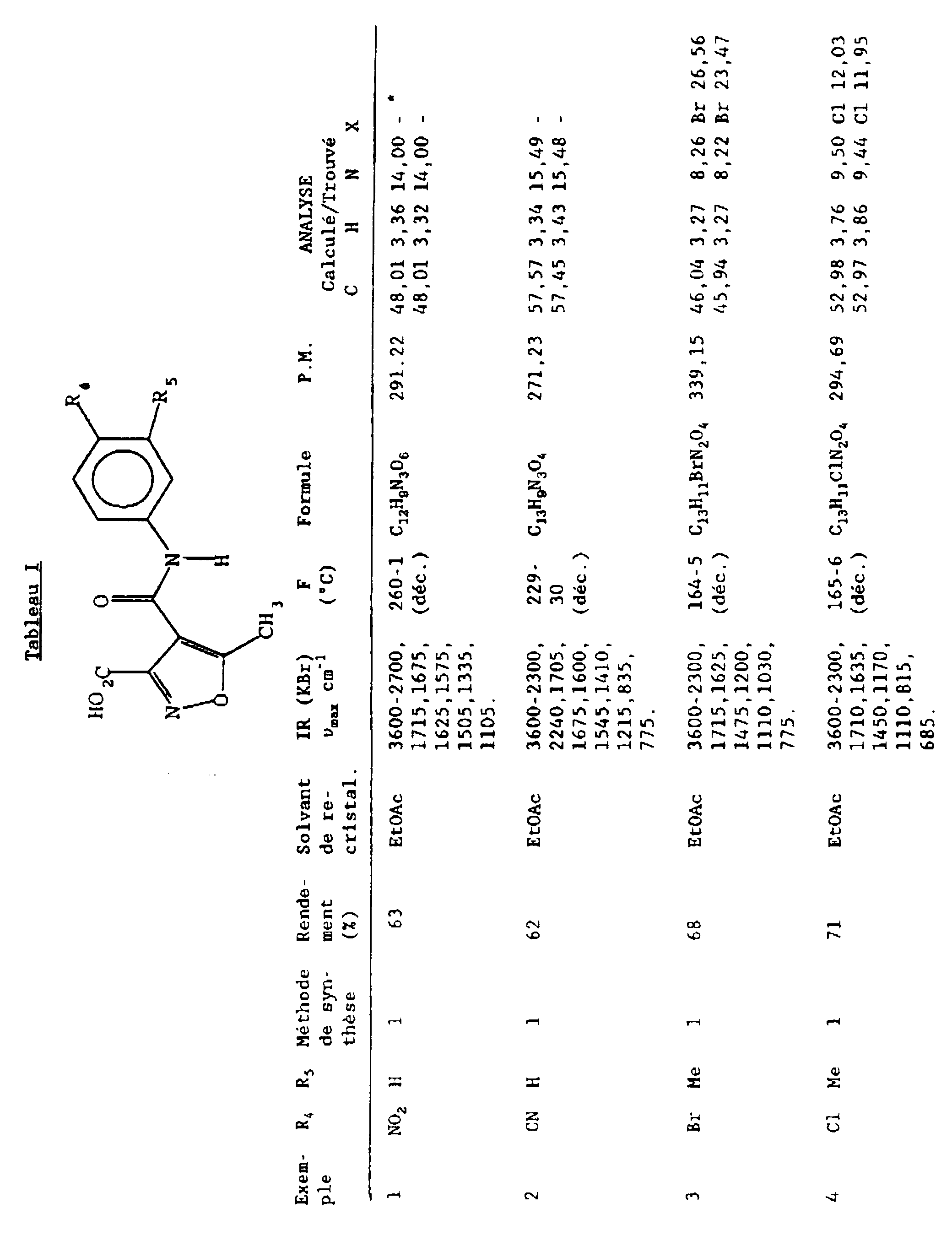

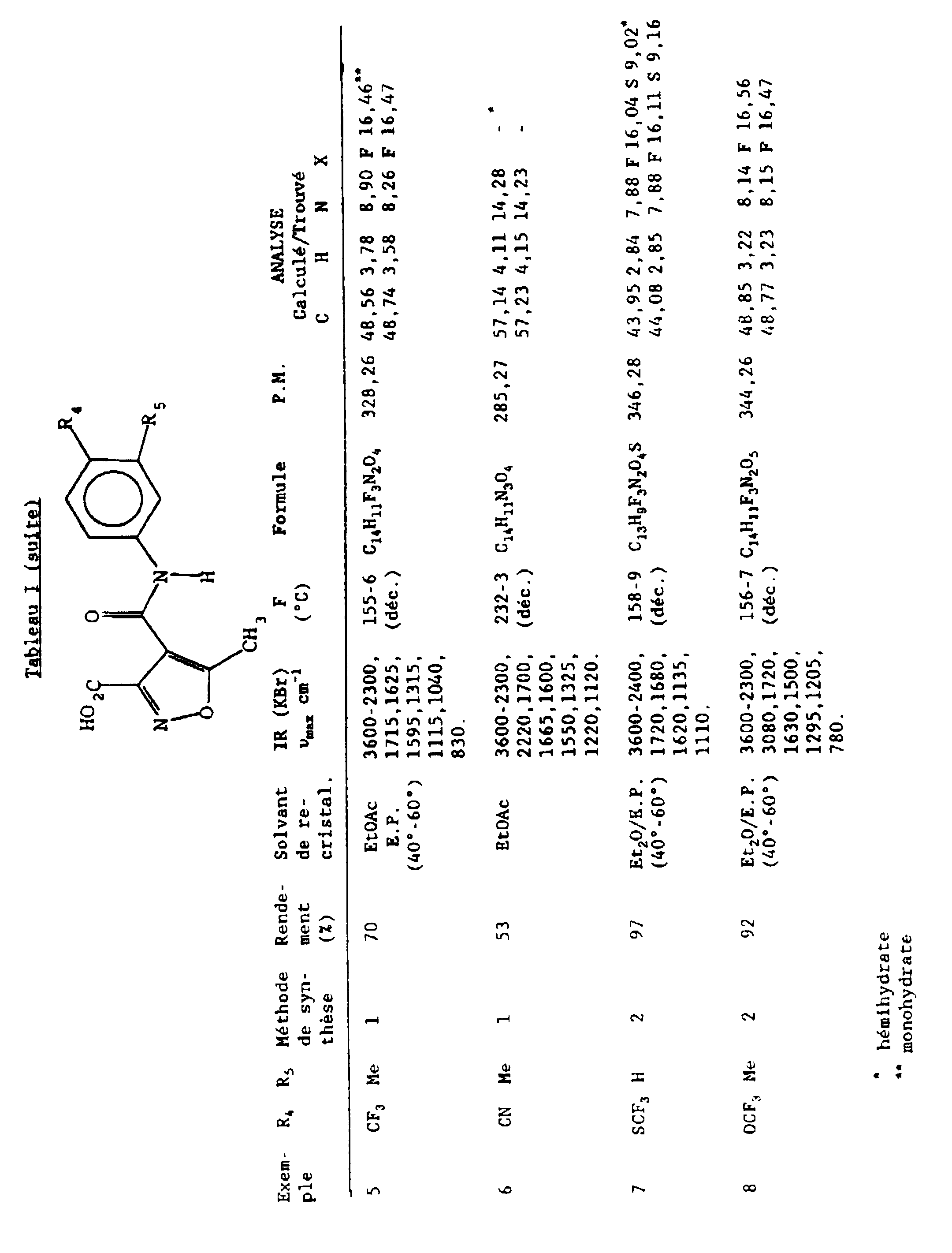

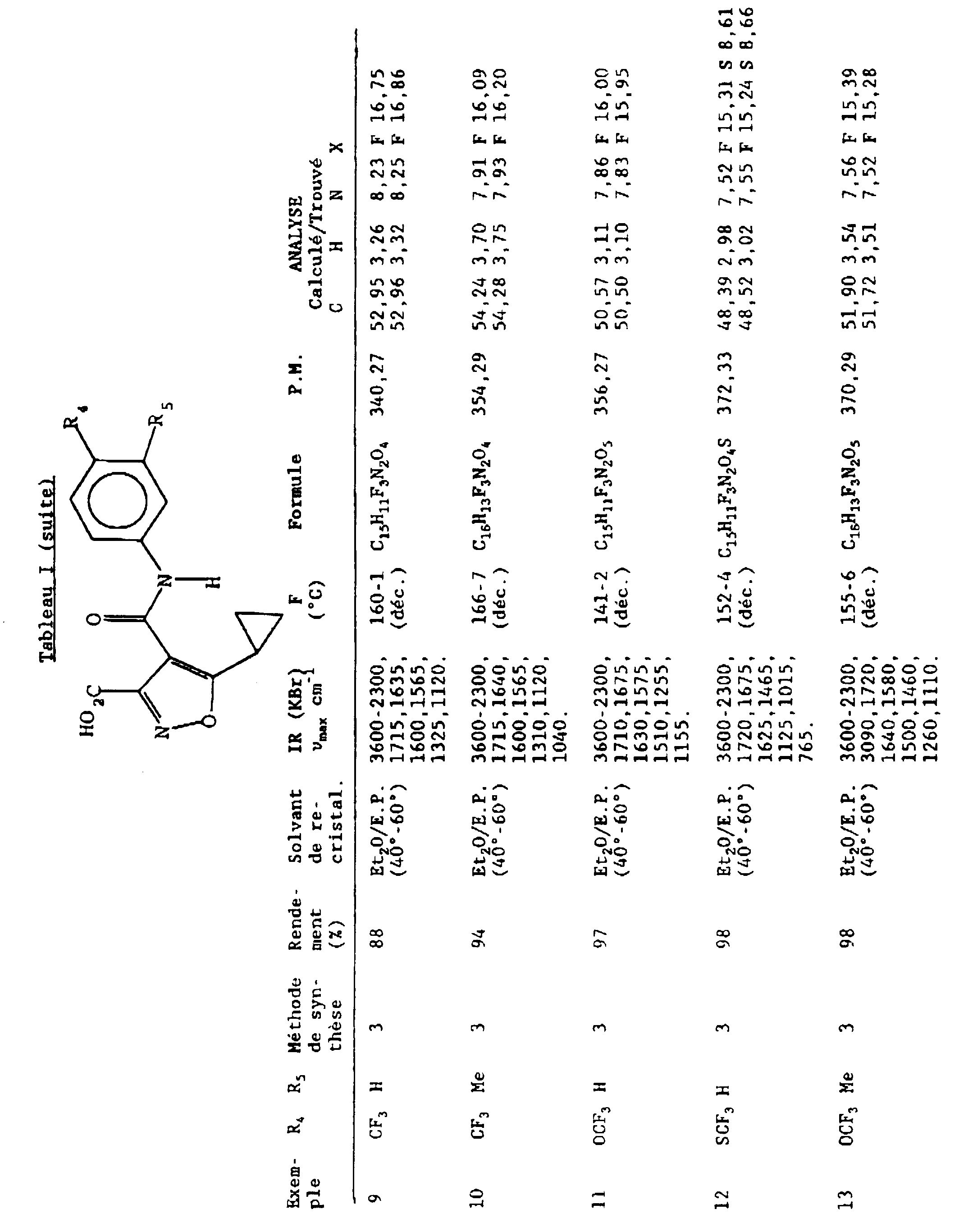

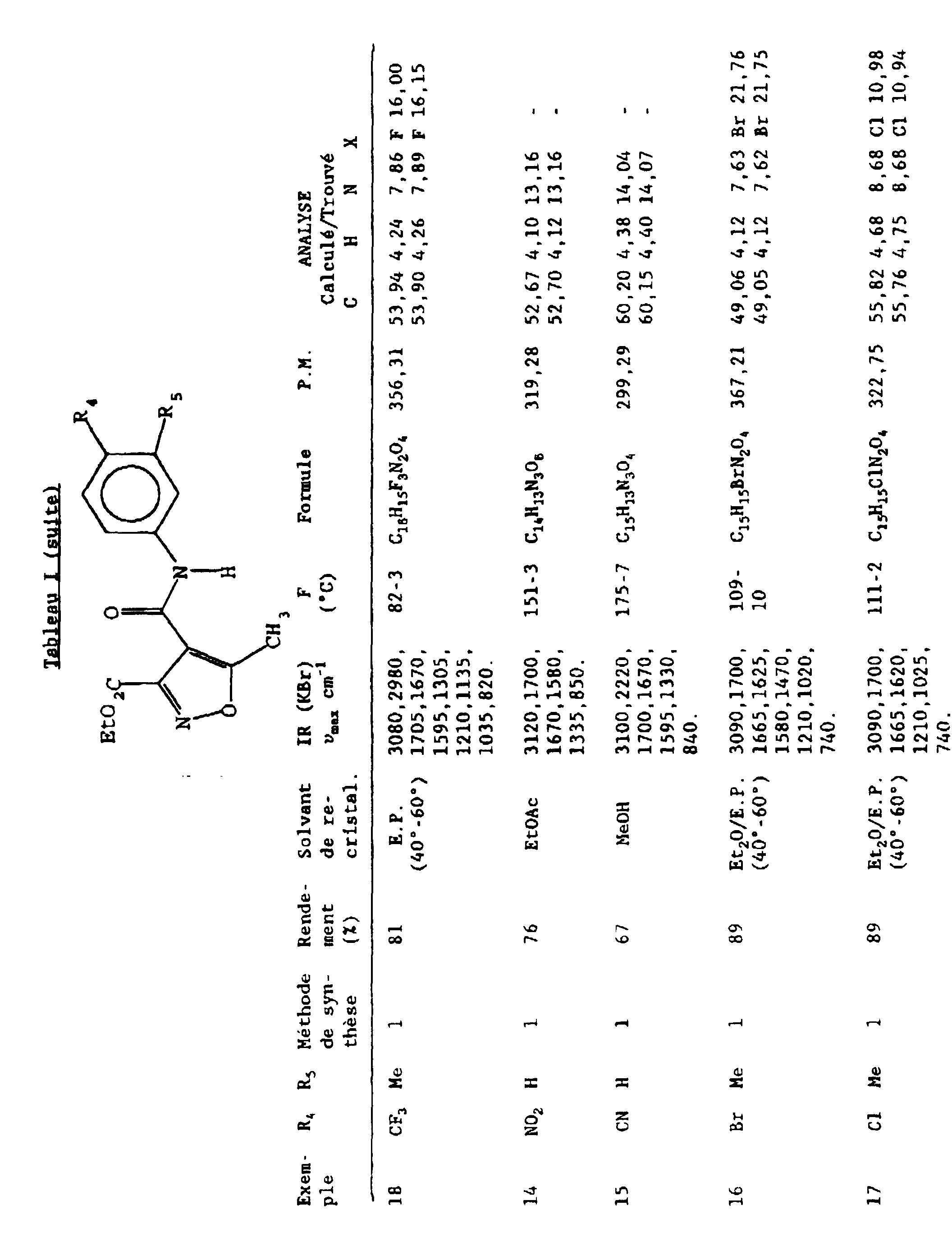

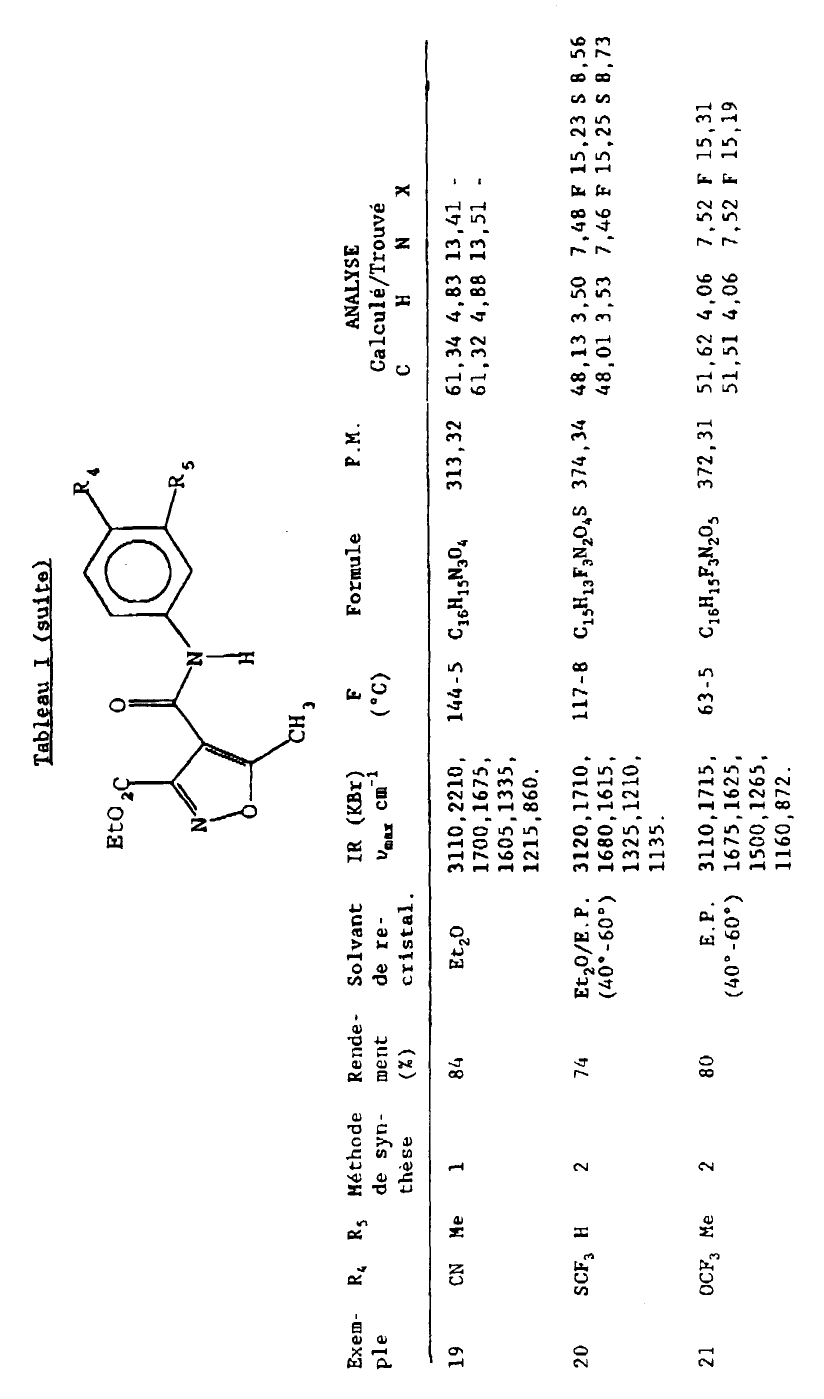

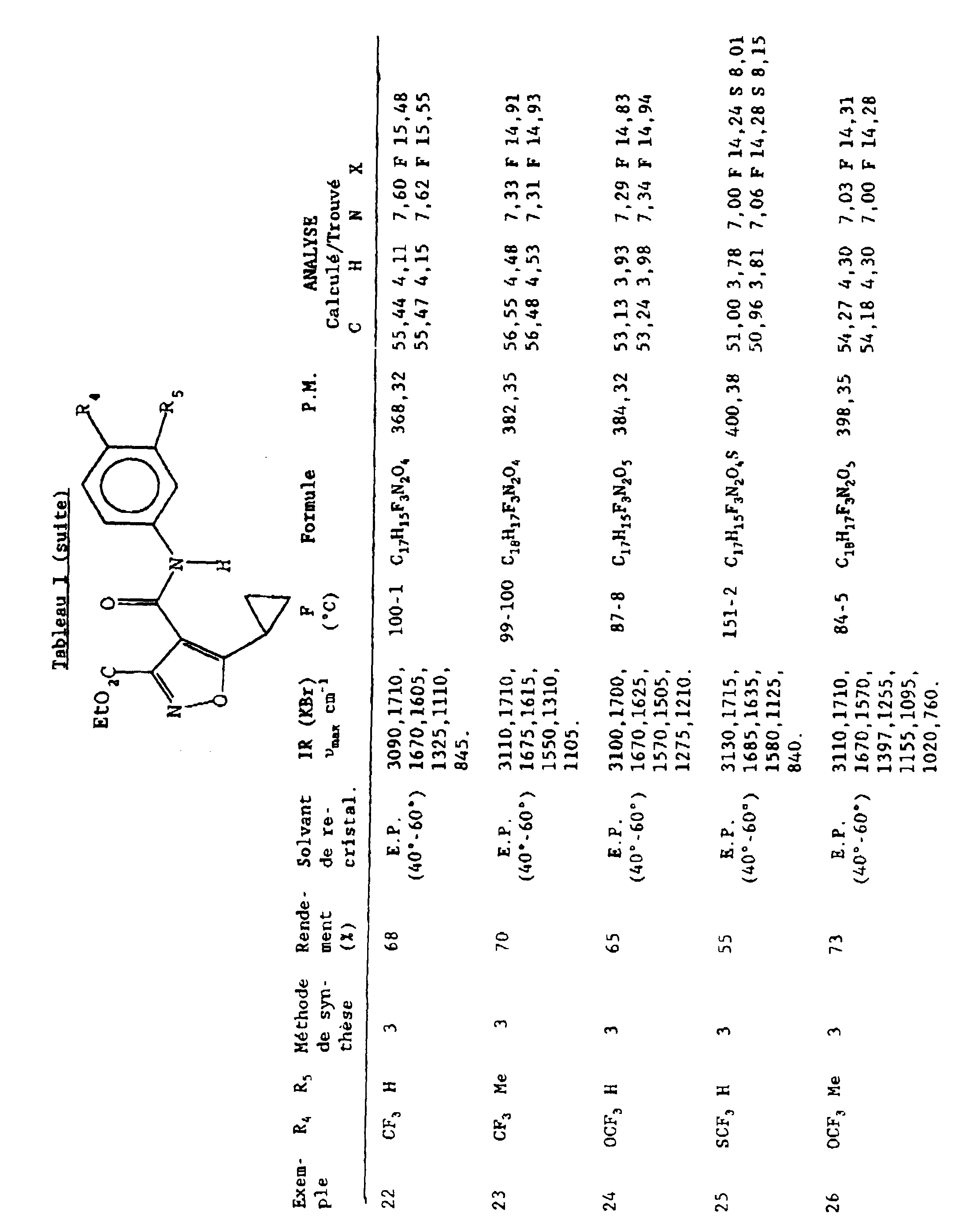

La méthode de synthèse 1 est représentée schématiquement de la manière suivante pour l'exemple 1:

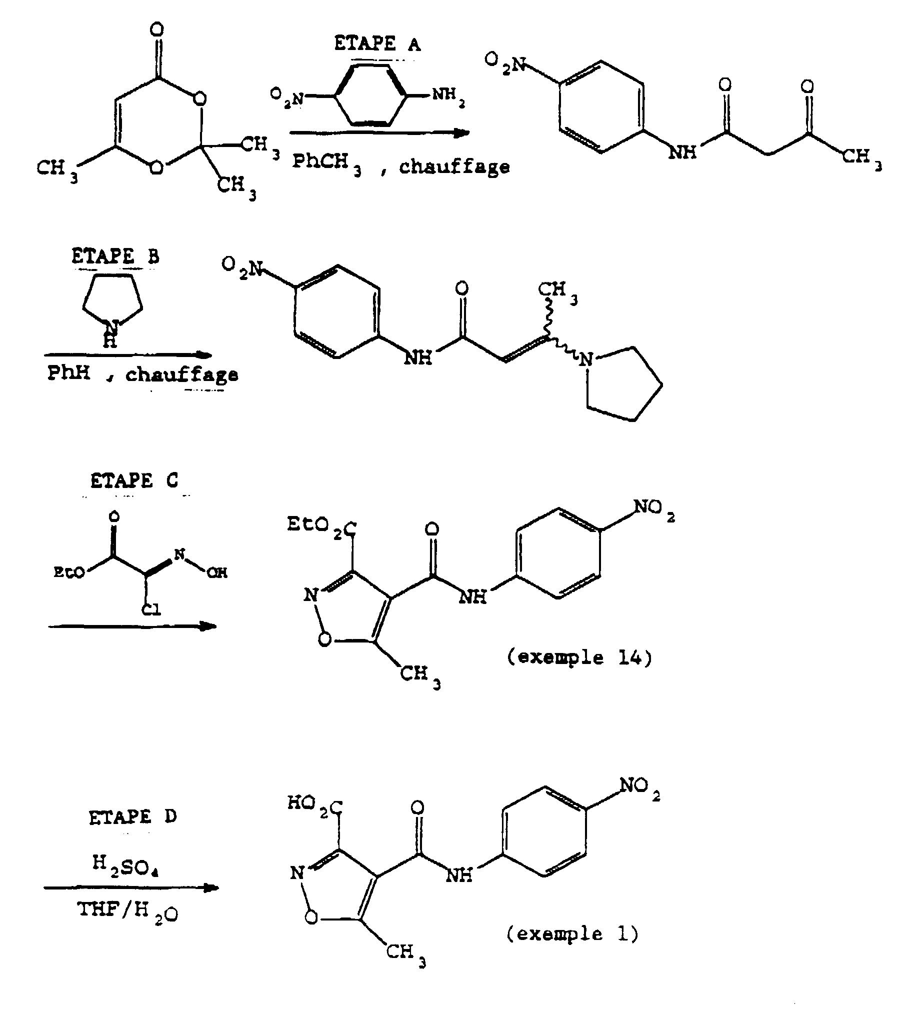

La méthode de synthèse 2 est représentée schématiquement de la manière suivante pour l'exemple 7:

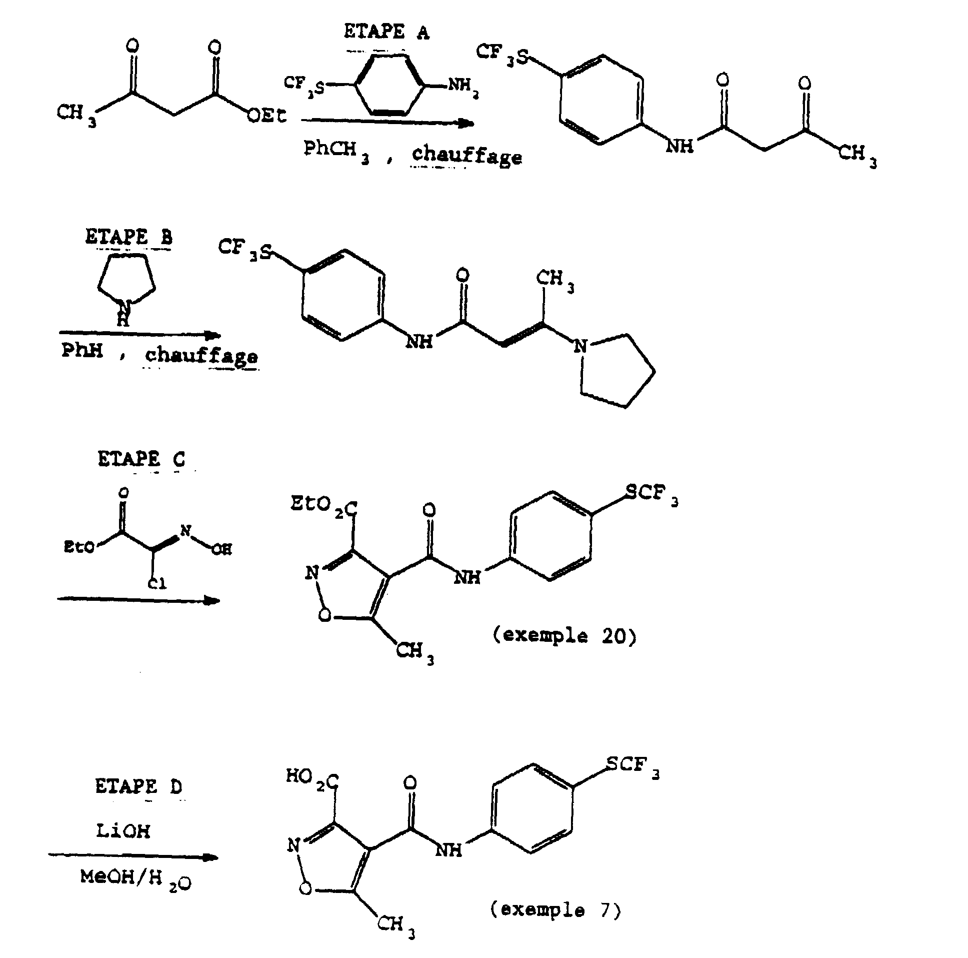

La méthode de synthèse 3 est représentée schématiquement de la manière suivante pour l'exemple 9:

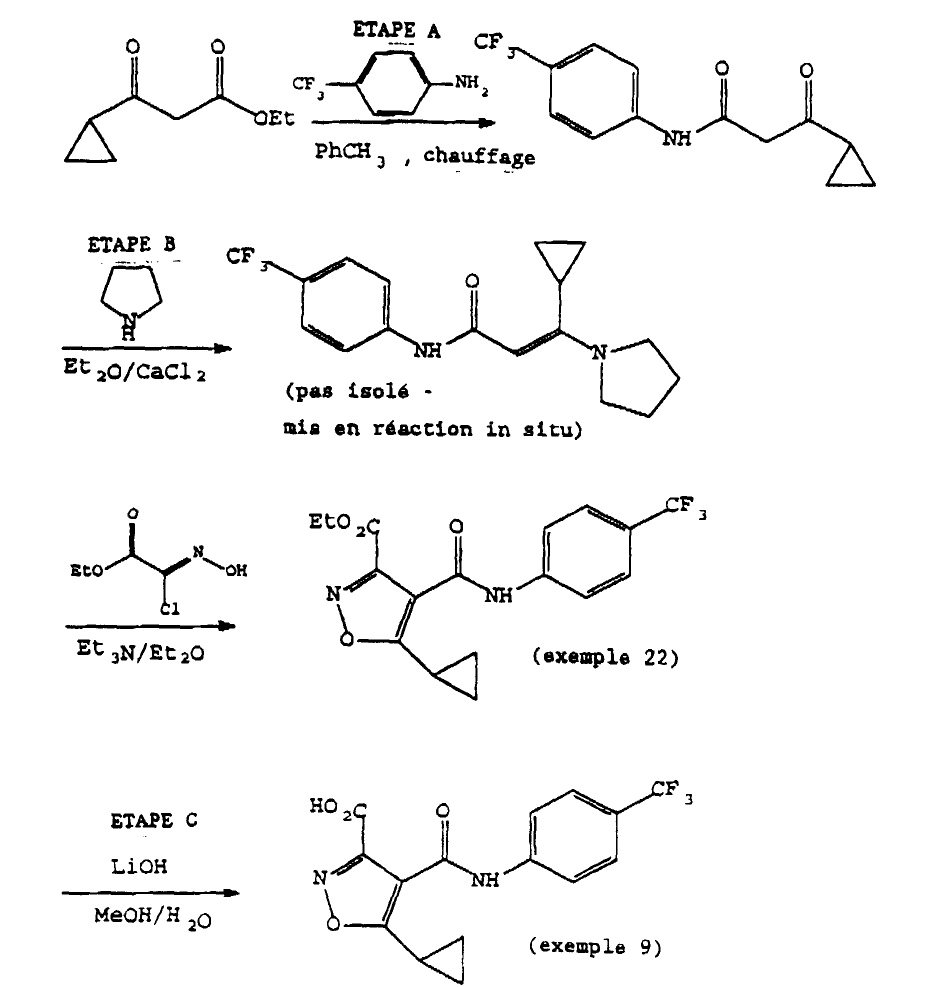

### DONNEES PHARMACOCINETIQUES

Une comparaison pharmacocinétique a été effectuée entre le composé selon l'invention de l'exemple 8, dans lequel R₅ représente un groupement méthyle et le composé connu correspondant dans lequel R₅ représente un atome d'hydrogène.

Chaque composé a été administré à des souris à une dose de 30 mg/kg p.o. et les concentrations plasmatiques du composé de base et du métabolite actif correspondant ont été déterminées à divers intervalles de temps suivant l'administration.

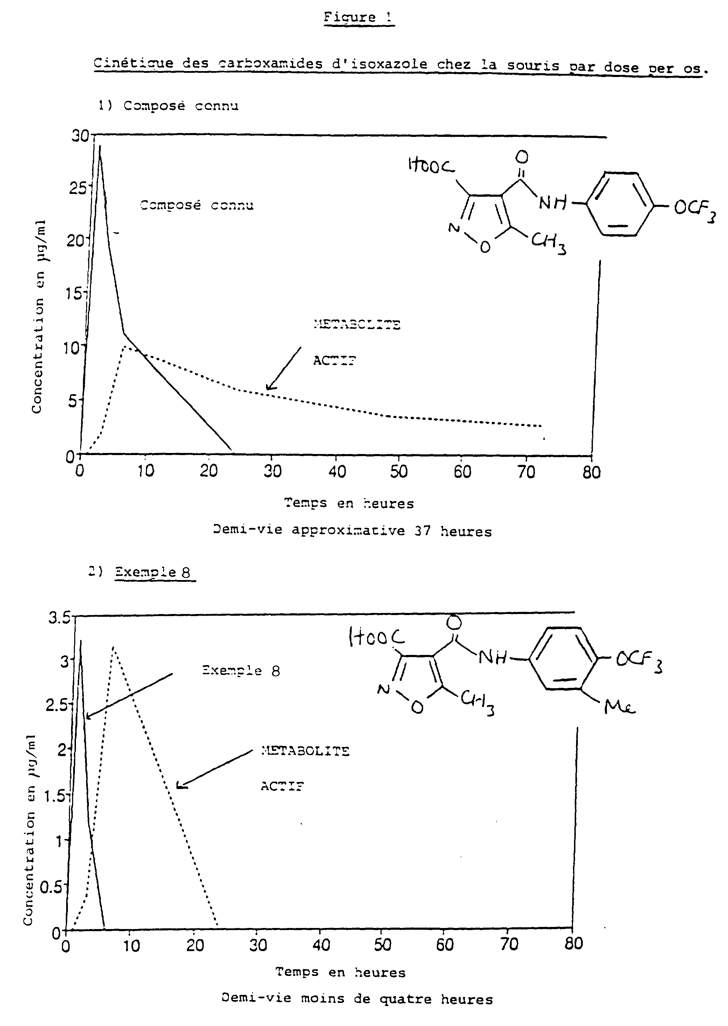

Les résultats sont présentés à la figure 1. Les résultats montrent que la demi-vie du métabolite actif du composé connu est voisine de 37 heures, le métabolite étant toujours décelable 72 heures après l'administration. Par contre, le composé de l'exemple 8 forme un métabolite actif avec une demi-vie inférieure à 4 heures, aucun métabolite n'étant décelable 24 heures après l'administration.

Ainsi, la présence du substituant méthyle dans le composé de l'exemple 8 se traduit clairement par une pharmacocinétique améliorée.

### ACTIVITES PHARMACOLOGIQUES

### Procédés d'essai biochimique

### Essai 1

### Oedème de carrageenan des pattes de rat (PO-R)

Une heure suivant l'administration par voie orale des composes à l'essai ou du véhicule témoin à des groupes de rats (n = 6-12, mâle CFHB, intervalle de poids 160-180 g) 1 mg de carrageenan, dissous dans 0,2 ml de liquide physiologique, est injecté dans la patte arrière droite.

Les pattes controlatérales reçoivent des injections de liquide physiologique témoin. Les réponses d'oedème des pattes sont contrôlées trois heures plus tard.

### Essai 2

Hypersensibilité de type retardé d'oedème des pattes de souris (DTH-M)

Des groupes de souris (n = 8-10, mâle CD-1, intervalle de poids 25-30 g) sont sensibilisés par injection sous-cutanée de 1 mg de sérum albumine bovine méthylée (MBSA) dans des volumes de 0,2 ml de liquide physiologique/émulsion d'adjuvant complet de Freund (FCA). Les groupes témoins négatifs reçoivent des injections de liquide physiologique/émulsion FCA. Les réponses DTH d'oedème des pattes sont contrôlées vingt-quatre heures après l'épreuve de la patte arrière droite par 0,1 mg de MBSA dans des volumes de 0,05 ml de liquide physiologique au jour sept suivant la sensibilisation.
Les pattes controlatérales reçoivent des injections de liquide physiologique témoin. Les composés à l'essai et les véhicules témoins sont administrés par voie orale chaque jour aux jours quatre, cinq, six et deux fois au jour sept, une heure avant et six heures après l'épreuve par MBSA.

### Essai 3

L'hypersensibilité de type retardé d'oedème des pattes de rat (DTH-R)

Des groupes de rats (n = 8-12, mâle CFHB, intervalle 160-180 g) sont sensibilisés par injection sous-cutanée à la base de la queue avec des volumes de 0,1 ml de FCA. Les groupes témoins négatifs reçoivent une injection d'adjuvant incomplet de Freund. Les réponses DTH d'oedème des pattes sont contrôlées vingt-quatre heures après l'épreuve des pattes arrières droites par 0,4 mg d'anti-gène d'extrait de Mycobactérium tuberculosis dans 0,2 volume de liquide physiologique au jour sept après la sensibilisation. Les pattes controlatérales reçoivent des injections témoins de liquide physiologique.

Les composés à l'essai sont administrés par voie orale chaque jour aux jours quatre, cinq, six et deux fois au jour sept, une heure avant et six heures après l'épreuve antigénique.

Les résultats de ces essais sont présentés dans le tableau II. Les doses sont données en unités mg/kg p.o.

**TABLEAU II**

| Exemple | Essai 1 | | Essai 2 | | Essai 3 | |
|---|---|---|---|---|---|---|
| | % Inhibition | Dose | % Inhibition | Dose | % Inhibition | Dose |
| 1 | -2 | 50 | 41 | 100 | 28 | 50 |
| 2 | -25 | 50 | 27 | 100 | -47 | 50 |
| 3 | -10 | 50 | 35 | 100 | -4 | 50 |
| 4 | -10 | 50 | 24 | 100 | 6 | 50 |
| 5 | 18 | 50 | 44 | 100 | 95 | 50 |
| | | | | | 44 | 10 |
| 6 | 5 | 50 | 5 | 100 | 25 | 50 |
| 7 | 1 | 50 | 14 | 100 | 73 | 50 |
| | | | | | 42 | 10 |
| 8 | 16 | 10 | 48 | 100 | 79 | 50 |
| | | | | | 34 | 10 |
| 9 | -24 | 10 | 72 | 100 | 86 | 50 |
| | | | | | 45 | 10 |
| 10 | 5 | 50 | 12 | 100 | 73 | 50 |
| 11 | -2 | 3 | 65 | 100 | Toxique | 50 |
| | | | 37 | 30 | 95 | 10 |
| | | | | | 65 | 3 |
| 12 | 11 | 10 | 36 | 100 | 75 | 10 |
| 13 | 5 | 10 | 52 | 100 | 47 | 10 |
| 18 | 23 | 50 | 3 | 100 | 50 | 50 |

## Revendications

1. Composés de formule (I): 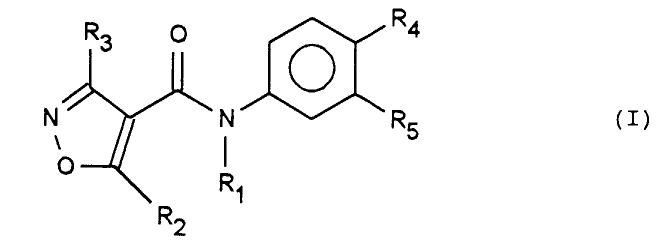 dans lesquels
R₁ représente un atome d'hydrogène ou un groupement alkyle comportant de 1 à 3 atomes de carbone;
R₂ représente un groupement alkyle comportant de 1 à 3 atomes de carbone ou un groupement cycloalkyle comportant de 3 à 6 atomes de carbone;
R₃ représente un groupement -COOH ou un groupement -COOR₆ dans lequel R₆ représente un groupement alkyle comportant de 1 à 3 atomes de carbone;
R₄ repésente un atome d'halogène, un groupement nitrile, un groupement nitro, un groupement -SCH₃, un groupement alkylcarbonyle en C₁₋₆, un groupement cycloalkylcarbonyle en C₃₋₆, un groupement -CX₃, un groupement -WCX₃, un groupement -(CH₂)ₙCX₃, un groupement -(CX₂)ₙCX₃, un groupement -W(CX₂)ₙCX₃ ou un groupement -W(CH₂)ₙCX₃,
dans lesquels X représente un atome d'halogène, W représente un atome d'oxygène ou de soufre, et n représente 1, 2 ou 3; et
R₅ représente un groupement alkyle comportant de 1 à 3 atomes de carbone ou un groupement cycloalkyle comportant de 3 à 6 atomes de carbone et leurs sels d'addition de base.

2. Composés selon la revendication 1, dans lesquels R₁ représente un atome d'hydrogène; R₂ représente un groupement méthyle ou un groupement cyclopropyle; R₄ représente un atome d'halogène, un groupement nitrile, un groupement nitro, un groupement -CF₃, un groupement -OCF₃ ou un groupement -SCF₃; et R₅ représente un groupement méthyle ou éthyle, ou un groupement cyclopropyle.

3. Composés selon la revendication 1 ou la revendication 2, dans lesquels R₄ représente un groupement -CF₃, un groupement -OCF₃ ou un groupement -SCF₃ et R₅ représente un groupement méthyle.

4. Composés selon la revendication 1 ou la revendication 2, caractérisés en ce qu'ils sont choisis parmi:
l'acide 5-méthyl-4-[N-(3-méthyl-4-trifluorométhyl)phényl]carbamoyl-3-isoxazolecarboxylique;
l'acide 5-méthyl-4-[N-(3-méthyl-4-trifluorométhoxy)phényl]carbamoyl-3-isoxazolecarboxylique;
l'acide 5-cyclopropyl-4-[N-(3-méthyl-4-trifluorométhoxy)phényl]carbamoyl-3-isoxazolecarboxylique; et leurs sels d'addition de base.

5. Procédé de préparation d'un composé de formule (I) selon la revendication 1, caractérisée en ce qu'il comprend la réaction d'un composé de formule (II) : 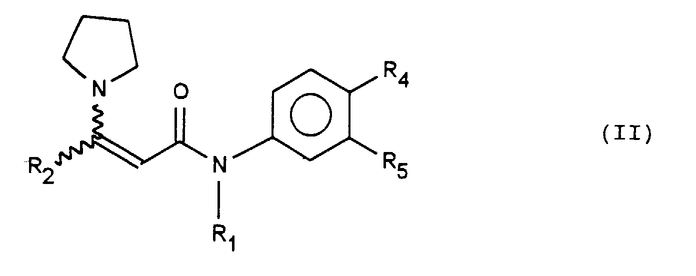 (dans lequel R₁, R₂, R₄ et R₅ sont tels que définis dans la revendication 1) avec un composé de formule (III): 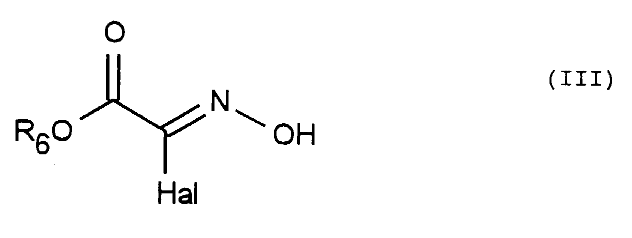 (dans lequel Hal représente un atome d'halogène et R₆ est tel que défini dans la revendication 1), pour préparer un composé de formule (I) dans lequel R₃ représente un groupement -COOR₆ et R₁, R₂, R₄, R₅ et R₆ sont tels que définis ci-dessus;
et ultérieurement, le cas échéant, la saponification du composé obtenu pour préparer un composé de formule (I) selon la revendication 1 dans lequel R₃ représente un groupement -COOH et R₁, R₂, R₄ et R₅ sont tels que définis ci-dessus.

6. Procédé selon la revendication 5, caractérisé en ce que le composé de formule (II) est préparé par réaction d'un composé de formule (IV) : 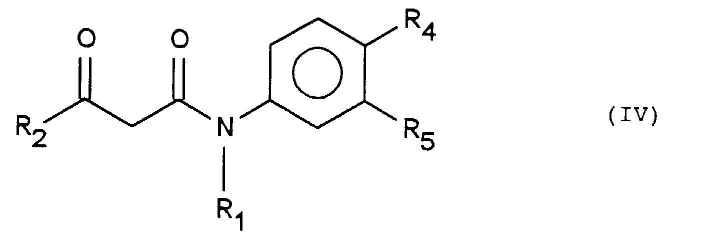 (dans lequel R₁, R₂, R₄ et R₅ sont tels que définis dans la revendication 1) avec la pyrrolidine.

7. Procédé selon la revendication 6, caractérisé en ce que l'on fait réagir in situ le composé de formule (II) avec le composé de formule (III).

8. Procédé selon la revendication 6 ou la revendication 7, caractérisé en ce que le composé de formule (IV) est préparé par réaction d'un composé de formule (V) : 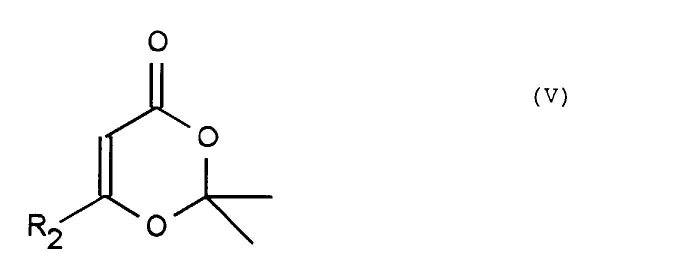 (dans lequel R₂ est tel que défini dans la revendication 1) avec un composé de formule (VI) : 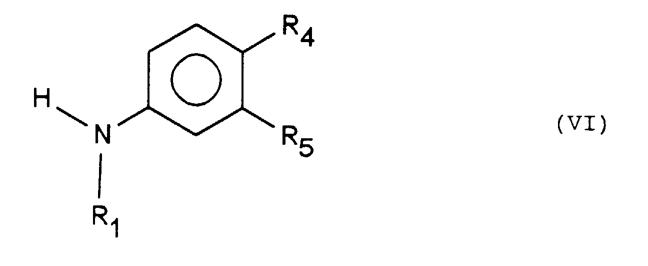 (dans lequel R₁, R₄ et R₅ sont tels que définis dans la revendication 1).

9. Procédé selon la revendication 6 ou la revendication 7, caractérisé en ce que le composé de formule (IV) est préparé par réaction d'un composé de formule (VII) :
R₂-COCH₂CO₂Et (VII)
(dans lequel R₂ est tel que défini dans la revendication 1) avec un composé de formule (VI) tel que défini dans la revendication 8.

10. Compositions pharmaceutiques comprenant à titre de principe actif au moins un composé de formule (I) selon l'une quelconque des revendications 1 à 4 ou son sel d'addition de base pharmacologiquement acceptable en association avec un ou plusieurs supports et/ou excipients pharmaceutiques.

11. Composés de formule (I) selon l'une quelconque des revendications 1 à 4 et leurs sels d'addition de base pharmacologiquement acceptables et compositions selon la revendication 10 pour une utilisation en tant que médicaments.

12. Composés de formule (I) selon l'une quelconque des revendications 1 à 4 et leurs sels d'addition de base pharmacologiquement acceptables et compositions selon la revendication 10 pour une utilisation dans le traitement de polyarthrite rhumatoïde et de maladies inflammatoires chroniques d'origine immunitaire ou non-immunitaire.

13. Composés de formule (II) tels que définis dans la revendication 5.

## Patentansprüche

1. Verbindungen der Formel (I) 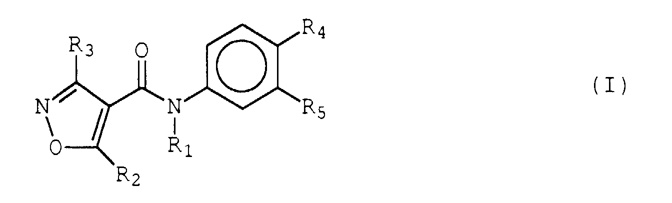 in der
R₁ ein Wasserstoffatom oder eine Gruppe Alkyl mit 1 bis 3 Kohlenstoffatomen darstellt;
R₂ eine Gruppe Alkyl mit 1 bis 3 Kohlenstoffatomen oder eine Gruppe Cycloalkyl mit 3 bis 6 Kohlenstoffatomen bedeutet;
R₃ eine Gruppe -COOH oder eine Gruppe -COOR₆ darstellt, worin R₆ eine Gruppe Alkyl mit 1 bis 3 Kohlenstoffatomen ist;
R₄ ein Halogenatom, eine Gruppe Nitril, eine Gruppe Nitro, eine Gruppe -SCH₃, eine Gruppe Alkylcarbonyl mit 1 bis 6 Kohlenstoffatomen, eine Gruppe Cycloalkylcarbonyl mit 3 bis 6 Kohlenstoffatomen, eine Gruppe -CX₃, eine Gruppe -WCX₃, eine Gruppe -(CH₂)ₙCX₃, eine Gruppe -(CX₂)ₙCX₃, eine Gruppe -W(CX₂)ₙCX₃ oder eine Gruppe -W(CH₂)ₙCX₃ bedeutet,
worin X ein Halogenatom ist, W ein Sauerstoffatom oder Schwefelatom darstellt und n 1, 2 oder 3 ist; und
R₅ eine Gruppe Alkyl mit 1 bis 3 Kohlenstoffatomen oder eine Gruppe Cycloalkyl mit 3 bis 6 Kohlenstoffatomen bedeutet,
sowie ihre Baseadditionssalze.

2. Verbindungen nach Anspruch 1, worin R₁ ein Wasserstoffatom ist; R₂ eine Gruppe Methyl oder eine Gruppe Cyclopropyl darstellt; R₄ ein Halogenatom, eine Gruppe Nitril, eine Gruppe Nitro, eine Gruppe -CF₃, eine Gruppe -OCF₃ oder eine Gruppe -SCF₃ bedeutet; und R₅ eine Gruppe Methyl oder Ethyl oder eine Gruppe Cyclopropyl darstellt.

3. Verbindungen nach Anspruch 1 oder Anspruch 2, worin R₄ eine Gruppe -CF₃, eine Gruppe -OCF₃ oder eine Gruppe -SCF₃ darstellt und R₅ eine Gruppe Methyl bedeutet.

4. Verbindungen nach Anspruch 1 oder Anspruch 2, dadurch gekennzeichnet, daß sie ausgewählt werden unter:
5-Methyl-4-[N-(3-methyl-4-trifluormethyl)-phenyl]-carbamoyl-3-isoxazolcarbonsäure;
5-Methyl-4-[N-(3-methyl-4-trifluormethoxy)-phenyl]-carbamoyl-3-isoxazolcarbonsäure;
5-Cyclopropyl-4-[N-(3-methyl-4-trifluormethoxy)-phenyl]carbamoyl-3-isoxazolcarbonsäure;
und ihren Baseadditionssalzen.

5. Verfahren zur Herstellung einer Verbindung der Formel (I) nach Anspruch 1, dadurch gekennzeichnet, daß es die Reaktion einer Verbindung der Formel (II) 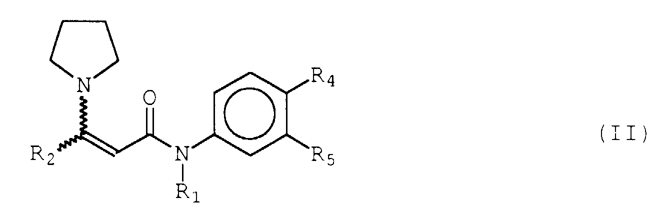 (in der R₁, R₂, R₄ und R₅ wie in Anspruch 1 definiert sind) mit einer Verbindung der Formel (III) 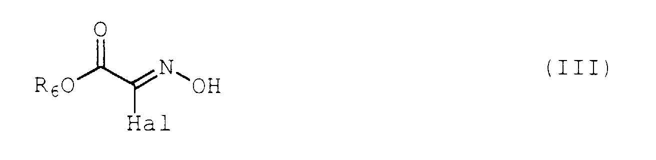 (in der Hal ein Halogenatom darstellt und R₆ wie in Anspruch 1 definiert ist), um eine Verbindung der Formel (I) herzustellen, in der R₃ eine Gruppe -COOR₆ ist und R₁, R₂, R₄, R₅ und R₆ wie oben definiert sind;
und gegebenenfalls später die Verseifung der erhaltenen Verbindung umfaßt, um eine Verbindung der Formel (I) nach Anspruch 1 herzustellen, in der R₃ eine Gruppe -COOH ist und R₁, R₂, R₄ und R₅ wie oben definiert sind.

6. Verfahren nach Anspruch 5, dadurch gekennzeichnet, daß die Verbindung der Formel (II) durch Reaktion einer Verbindung der Formel (IV) : 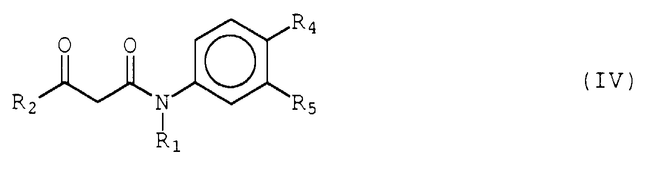 (in der R₁, R₂, R₄ und R₅ wie in Anspruch 1 definiert sind) mit Pyrrolidin hergestellt wird.

7. Verfahren nach Anspruch 6, dadurch gekennzeichnet, daß man die Verbindung der Formel (II) mit der Verbindung der Formel (III) in situ reagieren läßt.

8. Verfahren nach Anspruch 6 oder Anspruch 7, dadurch gekennzeichnet, daß die Verbindung der Formel (IV) durch Reaktion einer Verbindung der Formel (V) 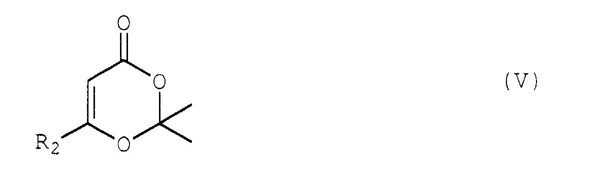 (in der R₂ wie in Anspruch 1 definiert ist) mit einer Verbindung der Formel (VI) 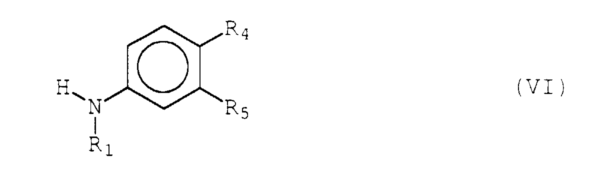 (in der R₁, R₄ und R₅ wie in Anspruch 1 definiert sind) hergestellt wird.

9. Verfahren nach Anspruch 6 oder Anspruch 7, dadurch gekennzeichnet, daß die Verbindung der Formel (IV) durch Reaktion einer Verbindung der Formel (VII)
R₂-COCH₂CO₂Et (VII)
(in der R₂ wie in Anspruch 1 definiert ist) mit einer Verbindung der Formel (VI), wie in Anspruch 8 definiert, hergestellt wird.

10. Pharmazeutische Zusammensetzungen, umfassend als Wirkstoff mindestens eine Verbindung der Formel (I) nach irgendeinem der Ansprüche 1 bis 4 oder ihr pharmakologisch akzeptables Baseadditionssalz in Assoziation mit einem oder mehreren pharmazeutischen Träger- und/oder Füllstoffen.

11. Verbindungen der Formel (I) nach irgendeinem der Ansprüche 1 bis 4 und ihre pharmakologisch akzeptablen Baseadditionssalze und Zusammensetzungen nach Anspruch 10 für eine Verwendung als Arzneimittel.

12. Verbindungen der Formel (I) nach irgendeinem der Ansprüche 1 bis 4 und ihre pharmakologisch akzeptablen Baseadditionssalze und Zusammensetzungen nach Anspruch 10 für eine Verwendung bei der Behandlung von rheumatoider Polyarthritis und von chronischen entzündlichen Erkrankungen immunitären oder nicht-immunitären Ursprungs.

13. Verbindungen der Formel (II) wie in Anspruch 5 definiert.

## Claims

1. Compounds of formula (I): 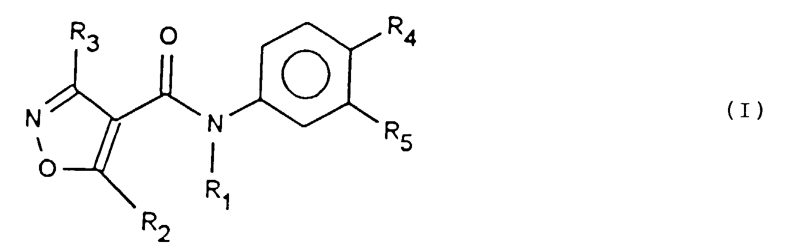 in which
R₁ represents a hydroden atom or an alkyl group containing 1 to 3 carbon atoms;
R₂ represents an alkyl group containing 1 to 3 carbon atoms or a cycloalkyl group containing 3 to 6 carbon atoms;
R₃ represents a -COOH group or a -COOR₆ group in which R₆ represents an alkyl group containing 1 to 3 carbon atoms,
R₄ represents a halogen atom, a nitrile group, a nitro group, a -SCH₃ group, a C₁₋₆ alkylcarbonyl group, a C₃₋₆ cycloalkylcarbonyl group, a -CX₃ group, a -WCX₃ group, a -(CH₂)ₙCX₃ group, a -(CX₂)ₙCX₃ group, a -W(CX₂)ₙCX₃ group or a -W(CH₂)ₙCX₃ group,
in which X represents a halogen atom, W represents a oxygen or sulphur atom, and n represents 1, 2 or 3; and
R₅ represents an alkyl group containing 1 to 3 carbon atoms or a cycloalkyl group containing 3 to 6 carbon atoms and their base addition salts.

2. Compounds according to claim 1, in which R₁ represents a hydrogen atom; R₂ represents a methyl group or a cyclopropyl group; R₄ represents a halogen atom, a nitrile group, a nitro group, a -CF₃ group, an -OCF₃ group or an -SCF₃ group; and R₅ represents a methyl or ethyl group, or a cyclopropyl group.

3. Compounds according to claim 1 or claim 2, in which R₄ represents a -CF₃ group, an -OCF₃ group or an -SCF₃ group and R₅ represents a methyl group.

4. Compounds according to claim 1 or claim 2, characterized in that they are chosen from:
5-methyl-4-[N-(3-methyl-4-trifluoromethyl)phenyl]carbamoyl-3-isoxazolecarboxylic acid;
5-methyl-4-[N-(3-methyl-4-trifluoromethoxy)phenyl]carbamoyl-3-isoxazolecarboxylic acid;
5-cyclopropyl-4-[N-(3-methyl-4-trifluoromethoxy)phenyl]carbamoyl-3-isoxazolecarboxylic acid; and their base addition salts.

5. Preparation process for a compound of formula (I) according to claim 1, characterized in that it comprises the reaction of a compound formula (II) : 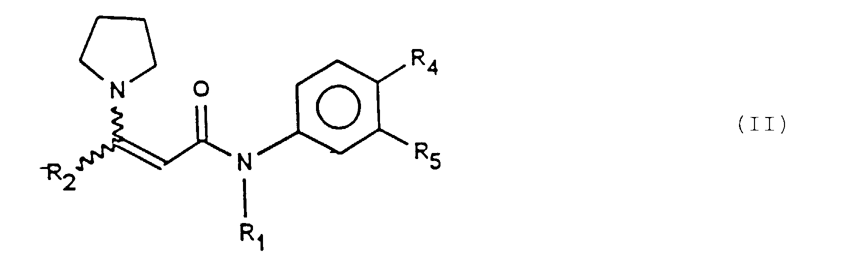 (in which R₁, R₂, R₄ and R₅ are as defined in claim 1) with a compound of formula (III): 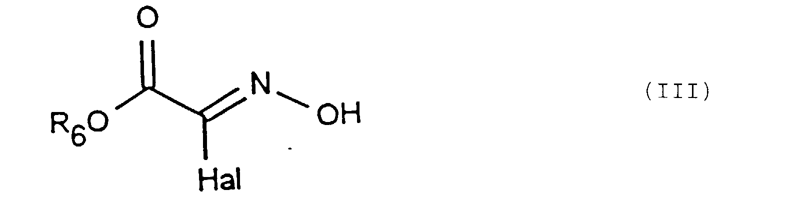 (in which Hal represents a halogen atom and R₆ is as defined in claim 1), for the preparation of a compound of formula (I) in which R₃ represents a -COOR₆ group and R₁, R₂, R₄, R₅ and R₆ are as defined above;
and subsequently, if appropriate, saponification of the compound obtained in order to prepare a compound of formula (I) according to claim 1, in which R₃ represents a -COOH group and R₁, R₂, R₄ and R₅ are as defined above.

6. Process according to claim 5, characterized in that the compound of formula (II) is prepared by the reaction of a compound of formula (IV) : 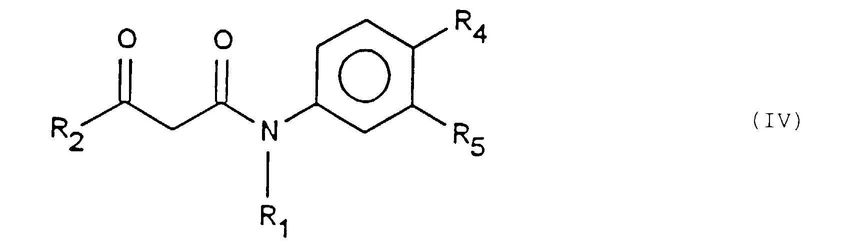 (in which R₁, R₂, R₄ and R₅ are as defined in claim 1) with pyrrolidine.

7. Process according to claim 6, characterized in that the compound of formula (II) is reacted in situ with the compound of formula (III).

8. Process according to claim 6 or claim 7, characterized in that the compound of formula (IV) is prepared by the reaction of a compound of formula (V) : 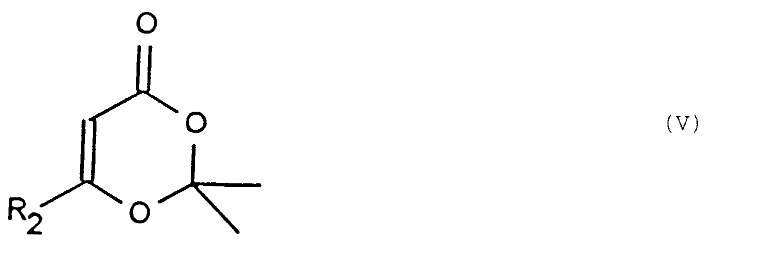 (in which R₂ is as defined in claim 1) with a compound of formula (VI) : 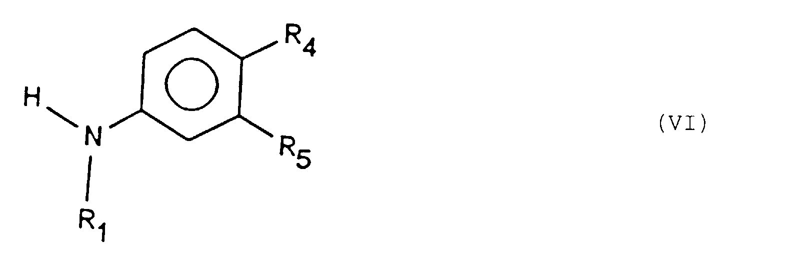 (in which R₁, R₄ and R₅ are as defined in claim 1).

9. Process according to claim 6 or claim 7, characterized in that the compound of formula (IV) is prepared by the reaction of a compound of formula (VII) :
R₂-COCH₂CO₂Et (VII)
(in which R₂ is as defined in claim 1) with a compound of formula (VI) as defined in claim 8.

10. Pharmaceutical compositions containing as active ingredient at least one compound of formula (I) according to any one of claims 1 to 4 or its pharmacologically acceptable base addition salts in combination with one or more pharmaceutical supports and/or excipients.

11. Compounds of formula (I) according to any one of claims 1 to 4 and their pharmacologically acceptable base addition salts and compositions according to claim 10 for use as medicaments.

12. Compounds of formula (I) according to any one of claims 1 to 4 and their pharmacologically acceptable base addition salts and compositions according to claim 10 for use in the treatment of rhumatoid polyarthritis and chronic inflammatory illnesses of immune or non-immune origin.

13. Compounds of formula (II) as defined in claim 5.
